# EUROPÄISCHE PATENTSCHRIFT

(11) **EP 1 084 765 B1**
(45) Veröffentlichungstag und Bekanntmachung des Hinweises auf die Patenterteilung: **15.02.2006**
(21) Anmeldenummer: 00120051.8
(22) Anmeldetag: 15.09.2000
(51) Int. Cl.: B05B 11/02, A61M 15/00, A61M 11/02

(54) **Vorrichtung zum ggf. zerstäubten Ausbringen eines insbesondere flüssigen Mediums**
Apparatus for dispensing, in particular for spraying fluid
Dispositif pour délivrer, notamment pour pulvériser un fluide

(30) Priorität: 15.09.1999 DE 19944211
(43) Veröffentlichungstag der Anmeldung: 21.03.2001
(60) Teilanmeldung: 05027267.3
(73) Patentinhaber: Ing. Erich Pfeiffer GmbH, 78315 Radolfzell (DE)
(72) Erfinder: Greiner-Perth, Jürgen, 78244 Gottmadingen (DE); Merk, Hans, 78343 Gaienhofen-Horn (DE)
(74) Vertreter: Patentanwälte Ruff, Wilhelm, Beier, Dauster & Partner

(56) Entgegenhaltungen:
- EP-A- 0 711 571
- DE-A- 19 749 514
- FR-A- 2 625 981
- US-A- 4 623 337
- US-A- 5 284 132
- US-A- 5 501 373

## Beschreibung

Aus der FR 88 00 454 ist eine Vorrichtung zum ggf. zerstäubten Ausbringen eines insbesondere flüssigen Mediums bekannt, bei dem das Medium in einem Spritzenzylinder aufbewahrt ist und das Medium über einen Ausbringungskanal ausgebracht werden kann. Hierzu ist im Spritzenzylinder ein Kolben angeordnet, der von einem Stößel beaufschlagbar ist, wobei der Kolben manuell betätigbar ist. Die manuelle Betätigung des Stößels erfolgt dabei über einen Betätigungsdrücker, der in einem Gehäuse geführt ist, das auch den Spritzenzylinder aufnimmt. Dabei sind an dem Gehäuse und an dem Betätigungsdrücker Führungsmittel vorgesehen, die die Betätigung des Betätigungsdrückers in der axialen Richtung des Spritzenzylinders begrenzen, wodurch der Gesamthub des Stößels im Spritzenzylinder in mehrere definierte Teilhübe unterteilt wird. Um am Ende eines Teilhubes den nächsten Teilhub ausführen zu können ist es erforderlich, den Betätigungsdrücker , gegenüber dem Spritzenzylinder bzw. gegenüber dem Gehäuse in dem der Spritzenzylinder angeordnet ist, zu verdrehen.

Daneben ist aus der EP 0 334 349 A1 eine Vorrichtung zum Ausbringen eines Mediums bekannt, bei der der Stößel eines Spritzenzylinders über einen Betätigungsdrücker betätigt wird. Der Betätigungsdrücker wird dabei in einem Gehäuse geführt, das als Aufnahme für den Spritzenzylinder dient. Dabei sind an dem Gehäuse Führungsnocken ausgebildet, die in einer am Betätigungsdrücker ausgeformten Kulissenbahn geführt werden. Die Kulissenbahn ist so geformt, daß der Betätigungsweg des Betätigungsdrückers in mehrere Teilhübe unterteilt wird. Dies geschieht entweder dadurch, daß die Kulissenbahn treppenförmig verläuft, so daß zwischen zwei Teilhüben des Betätigungsdrückers ein Verdrehen des Betätigungsdrückers gegenüber dem Gehäuse erforderlich ist oder aber indem die Kulissenbahn überdrückbare Raststellen aufweist, die die in der Kulissenbahn geführte Nocke in einer Endlage eines Teilhubes so lange halten, bis die Betätigungskraft eine Haltekraft übersteigt. In dem letzteren Falle kann die Kulissenbahn auch linear ausgebildet sein.

Die EP 0 711 571 A1 zeigt einen Zerstäuber gemäß dem Oberbegriff des Anspruchs 1, wobei dessen Kolbenstößel eine Endplatte aufweist, die sich auf einer gewölbten, strukturierten Gummimembran abstützt, die in zwei Stufen einknicken kann. Damit werden zwei Austragshübe abgegrenzt. Bei entsprechender Betätigungskraft kann diese jedoch überdrückt werden, so dass die Gefahr besteht, dass beide Teilhübe unmittelbar nacheinander erfolgen. Für pharmazeutische Zwecke ist dies unter Umständen gefährlich.

Auch bei der US 5 501 373 ist eine solche Überdrückungsgefahr gegeben. Dort sind auf einem Stößel zwei elastische Ringe angeordnet, die bei der Betätigung aus ihren Rasten geschoben werden.

Die FR 2 625 981 beschreibt einen Zerstäuber, bei dem der als Betätigungsdrücker dienende Zylinder Kontakt mit Nocken im Inneren einer Hülse hat, um den Betätigungsweg zu begrenzen. Für einen neuen Teilhub muss eine manuelle Verdrehung zwischen Zylinder und Hülse stattfinden.

Die DE 197 49 514 A1 zeigt einen Zwei-Komponenten-Zerstäuber, bei dem ein Pulver mit einer Flüssigkeit in einem ersten Betätigungsschritt gemischt und danach die Mischung ausgegeben wird. Es ist dort ein abreißbarer Ring vorgesehen, der einen Druckpunkt schafft, also nur durch eine ausreichende Betätigungskraft zu überwinden ist.

Die US 5 284 132 zeigt einen Zerstäuber, der, ähnlich wie die EP 0 334 349 A1, eine treppenartige Hubbegrenzung durch das Zusammenwirken eines stufigen Schlitzes mit einem Vorsprung aufweist.

Bei Vorrichtungen zum Ausbringen von Medien, bei denen ein Verdrehen des Betätigungsdrückers bzgl. dem Gehäuse erforderlich ist, um aufeinanderfolgende Teilhübe durchzuführen, ist es nachteilig, daß die Möglichkeit einer Einhandbetätigung nicht besteht. Zum Verdrehen des Betätigungsdrückers bzgl. dem Gehäuse ist es erforderlich, mit beiden Händen die Vorrichtung zu halten. Jedoch haben die gestuften Führungen den Vorteil, daß ein versehentliches aneinandergereihtes Durchführen zweier Teilhübe in einer Bewegung nicht möglich ist. Die Ausführung mit einer linearen Kulissenbahn und durch Überwindung einer Haltekraft überdrückbare Raststellen zur Unterteilung der Ausbringung des Mediums in mehrere Teilhübe, eröffnet zwar die Möglichkeit der Einhandbetätigung, es besteht hier aber die Gefahr, daß unbeabsichtigt mehrere Teilhübe in ununterbrochener Betätigung hintereinander ausgeführt werden.

### AUFGABE UND LÖSUNG

Aufgabe der Erfindung ist es, eine einhandbetätigbare Vorrichtung zum Ausbringen eines flüssigen Mediums zu schaffen, bei der sichergestellt ist, daß Teilhübe nicht unbeabsichtigt ununterbrochen hintereinander durchgeführt werden.

Diese Aufgabe wird durch Vorrichtungen gemäß den unabhängigen Ansprüchen gelöst.

Die Vorrichtung geht davon aus, dass ein Betätigungsdrücker an dem Stößel angreift, der den in dem Spritzenzylinder verschiebbaren Kolben betätigt, wobei der Betätigungsweg des Betätigungsdrückers zwischen einer Betätigungsausgangs- und Betätigungsendlage so begrenzt ist, dass der daraus resultierende Ausbringungshub des Kolbens dem Maß entspricht, das für das Ausbringen einer Teilcharge benötigt wird.

Gemäß vorteilhaften Ausbildungen der Vorrichtung wird eine Rückstellfeder zum Zurückführen des Betätigungsdrückers aus der Betätigungsendlage in die Betätigungsausgangslage vorgesehen. An dem auf den Kolben des Spritzenzylinders einwirkenden Stößel sind Mitnehmer angeformt, die lediglich in Richtung des Ausbringungshubes eine kraftschlüssige Verbindung zwischen Betätigungsdrücker und Stößel herstellen. Zusätzlich können gemäß vorteilhafter Ausgestaltung an dem Stößel auch Rastmittel angeformt sein, die in Betätigungsausgangslage an einer Rastkante anliegen und die durch Überwinden einer Mindestbetätigungskraft des Betätigungsdrückers überdrückbar sind. Besonders vorteilhaft kann es sein, daß die Mitnehmer derart ausgebildet sind, daß die Mitnehmer eines Ausbringungshubes als Rastmittel eines nachfolgenden Ausbringungshubes dienen.

Gemäß einer Ausgestaltung der Erfindung sind die Mitnehmer als Verdickungen des Stößels ausgebildet, besonders vorteilhaft ist es, den Abschnitt des Stößels, der als Mitnehmer ausgebildet ist, als Kegelstumpf auszubilden, wobei die gedachte Spitze des Kegelstumpfes in Richtung des Kolbens des Spritzenzylinders liegt. Weitere vorteilhafte Ausgestaltungen der Vorrichtung zum Ausbringen eines Mediums können den weiteren Unteransprüchen entnommen werden.

Eine zweite Vorrichtung zum Ausbringen eines Mediums wird ebenfalls aus einem Spritzenzylinder gebildet, in dem ein Kolben angeordnet ist, der über einen Stößel betätigbar ist. Hierzu liegt ein Gleitschuh an dem Stößel in einem Bereich außerhalb des Spritzenzylinders an und der Stößel weist wenigstens einen Bund auf, wobei in Betätigungsendlage eines der Ausbringer einer Teilcharge entsprechenden Betätigungshubes der Bund in Anlage mit dem Gleitschuh gelangt.

Gemäß vorteilhafter Ausgestaltung dieser Ausführungsform wird der Gleitschuh in Betätigungsendlage am Spritzenzylinder abgestützt. Eine weitere vorteilhafte Ausgestaltung ist darin zu sehen, daß der Bund durch eine Durchmesservergrößerung des Stößels gebildet wird. Weitere vorteilhafte Ausgestaltungen dieser Ausführungsform befassen. Weitere vorteilhafte Ausgestaltungen dieser Ausführungsform befassen sich mit der Anlenkung des Gleitschuhs.

Weitere Unteransprüche befassen sich mit vorteilhaften Ausgestaltungen beider Ausführungsformen.

Die vorstehenden und weitere Merkmale gehen außer aus den Ansprüchen auch aus der Beschreibung und den Zeichnungen hervor, wobei die einzelnen Merkmale jeweils für sich allein oder zu mehreren in Form von Unterkombinationen bei einer Ausführungsform der Erfindung und auf anderen Gebieten verwirklicht sein und vorteilhafte sowie für sich schutzfähige Ausführungen darstellen können, für die hier Schutz beansprucht wird. Die Unterteilung der Anmeldung in einzelne Abschnitte sowie Zwischen-Überschriften beschränken die unter diesen gemachten Aussagen nicht in ihrer Allgemeingültigkeit.

### KORZBESCHREIBUNG DER ZEICHNUNGEN

Ausführungsbeispiele der Erfindung sind in den Zeichnungen dargestellt und werden im folgenden näher erläutert. In den Zeichnungen zeigen:
- Fig. 1: in Schnittdarstellung ein erstes Ausführungsbeispiel einer Vorrichtung, bei der der Betätigungsweg des Betätigungsdrückers auf das für das Ausbringen einer Teilcharge benötigten Hub begrenzt ist;
- Fig. 2: den Querschnitt durch ein zweites Ausführungsbeispiel mit begrenztem Betätigungsweg eines Betätigungsdrückers und
- Fig. 3: die Schnittdarstellung eines Ausführungsbeispiels, bei dem der Weg des Stößels durch an Stößel anliegende Gleitschuhe begrenzt wird.

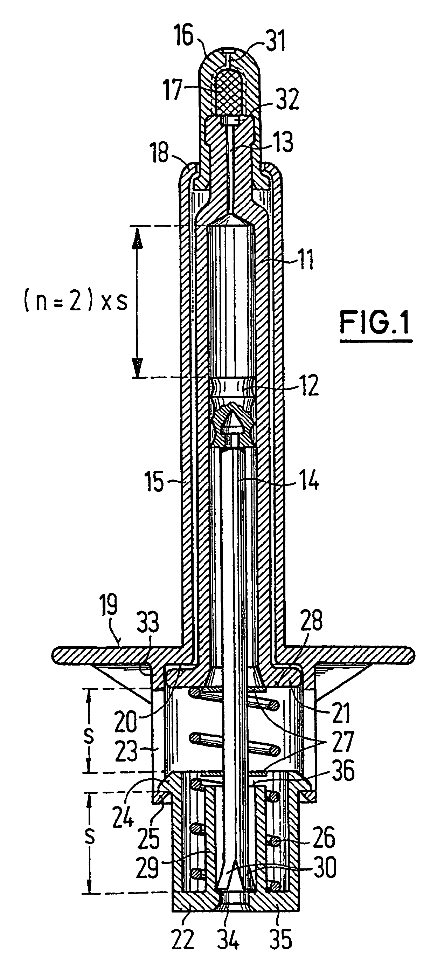

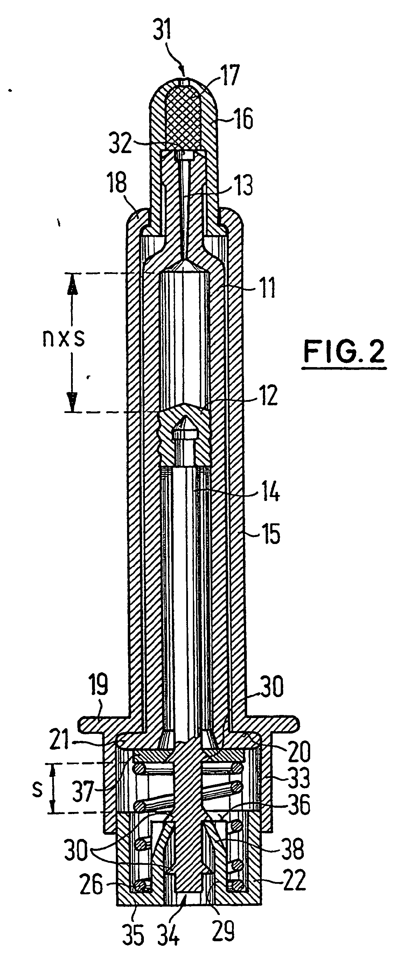

### BESCHREIBUNG DER AUSFÜHRUNGSBEISPIELE NACH DEN FIG. 1 UND 2

Die Fig. 1 zeigt den Querschnitt durch eine Vorrichtung zum ggf. zerstäubten Ausbringen eines insbesondere flüssigen Mediums. Das auszubringende Medium befindet sich in dem Spritzenzylinder 11, in dem der Kolben 12 in axialer Richtung des Spritzenzylinders verschiebbar gelagert ist. Der Spritzenzylinder 11 mündet in dem Ausbringungskanal 13, durch den hindurch das Medium ausgebracht wird, wenn über den Stößel 14 durch manuelle Betätigung der Kolben 12 im Spritzenzylinder 11 in Richtung auf den Ausbringungskanal 13 hin verschoben wird.

Auf der Seite des Ausbringungskanals 13 wird über die Überwurfkante 18 des Gehäuses 15 eine Kappe 16 gehalten, in die der Ausbringungskanal 13 des Spritzenzylinders 11 mündet. Die Kappe 16 weist an ihrer dem Ausbringungskanal 13 abgewandten, vorderen Spitze eine Düse 31 als Austrittsöffnung für das auszubringende Medium auf. Zwischen Kappe 16 und Mündung 32 des Ausbringungskanals 13 befindet sich das Dichtelement 17. Das Dichtelement 17 stellt eine hermetische Abdichtung zwischen Düse 31 und Mündung 32 des Ausbringungskanals 13 zumindest solange dar, wie die Vorrichtung nicht zum ersten mal betätigt wurde. Dadurch wird zuverlässig eine Verkeimung oder ein Luftzutritt zu dem in dem Spritzenzylinder 11 aufbewahrten auszubringenden Medium sichergestellt. Dies ist besonders dann wichtig, wenn es sich bei dem auszubringenden Medium um ein steril aufzubewahrendes Arzneimittel handelt. Dies könnten beispielsweise Medikamente gegen Migräne oder aber Impfseren sein, die dem Patienten über die Nase auf die Nasenschleimhäute appliziert werden, da sie dort besonders gut vom Patienten absorbiert werden können.

Das Dichtelement 17 kann dabei entweder so ausgebildet sein, daß es bei der ersten Betätigung, also dem ersten Teilhub, irreversibel einen Kanal zwischen Mündung 32 des Ausbringungskanals 13 und der Düse 31 der Kappe 16 freigibt. Es ist aber auch möglich, das Dichtelement 17, beispielsweise aus einer elatischen Masse, so auszubilden, daß der Kanal zwischen Mündung 32 und Düse 31 sich jeweils nur für die Dauer des Teilhubes öffnet und anschließend sofort wieder verschließt. Letztere Ausbildung hat den Vorteil, daß weiterhin keine Luft über die Düse 31 zum Ausbringungskanal 13 gelangen kann.

Am dem Ausbringungskanal 13 gegenüberliegenden Ende weist der Spritzenzylinder 11 eine Aufweitung 21 auf, die sich an der Anlagefläche 20 des Gehäuses 15 abstützt. Die Anlagefläche 20 wird durch eine radiale Aufweitung des Gehäuses 15, die vorteilhafterweise gleichzeitig als Fingeranlage 19, zur Abstützung des Gehäuses 15 in der Hand des Benutzers während der Betätigung der Vorrichtung dient, gebildet. Dadurch ist gleichzeitig sichergestellt, daß die Fingeranlage 19 schon vor dem ersten Teilhub - vom Ausbringungskanal 13 her gesehen - hinter dem Kolben 12 liegt, wodurch eine stabile und betätigungssichere Handhabung der Vorrichtung vorteilhafterweise erreicht wird.

Die Aufweitung 21 des Spritzenzylinders 11 umgebend, ragt der Schaft 33 des Gehäuses 15 nach hinten ab. In dem Schaft 33 befinden sich Führungsschlitze 23, in die Rastnasen 24, die an dem Betätigungsdrücker 22 angeformt sind, eingreifen. Des weiteren weist der Schaft 33 an seinem hinteren Ende einen die Führungsschlitze 23 abschließenden Abschlußring 25 auf. Der Betätigungsdrücker 22 ist in seiner Außenkontur napfförmig ausgebildet und durch seine Rastnasen 24 in dem Schaft 33 axial verschiebbar gehalten. Der mögliche Betätigungsweg s des Betätigungsdrückers 22 zwischen seiner dargestellten Betätigungsausgangslage und der Betätigungsendlage wird durch die freie Länge der Führungsschlitze 23 in dem Schaft 33 des Gehäuses 15 festgelegt. In Betätigungsendlage liegt der Betätigungsdrücker 22 mit seiner Vorderkante an der Aufweitung 21 des Spritzenzylinders 11 an.

Zwischen der Aufweitung 21 des Spritzenzylinders 11 und dem Betätigungsdrücker 22 ist die Rückstellfeder 26 angeordnet. Die Rückstellfeder 26 hat zwei Funktionen. Durch ihre Vorspannung erzeugt sie eine zwischen Betätigungsdrücker 22 und Spritzenzylinder 11 wirkende Kraft, so daß einerseits der Betätigungsdrücker 22 des Gehäuses 15 in seiner Betätigungsausgangslage gehalten wird, andererseits wird auch der Spritzenzylinder 11 gegen axiales Verschieben im Gehäuse 15 gesichert.

Der Stößel 14 reicht vom Kolben 12 bis zu der Bodenfläche 35 des Betätigungsdrückers 22. An dem Stößel 14 sind Rastmittel 27 angeformt, die jeweils um den Betätigungsweg s voneinander beabstandet sind und die in Betätigungsausgangslage eines Teilhubes an der Rastkante 28, die vorzugsweise durch die Aufweitung 21 des Spritzenzylinders 11 gebildet wird, anliegen. Die Rastmittel 27 bilden im Zusammenwirken mit der Rastkante 28 einen der Betätigung des Betätigungsdrückers 22 entgegenwirkenden Druckpunkt, wodurch vorzugsweise sichergestellt wird, daß bei jeder Betätigung des Betätigungsdrückers 22 an ihm eine derartige Betätigungskraft aufgebracht wird, daß er immer in einer durchgehenden, ununterbrochenen Betätigung um seinen gesamten Betätigungsweg von der Betätigungsausgangslage in die Betätigungsendlage verbracht wird. An seinem, dem Kolben 12 abgewandten, hinteren Ende weist der Stößel 14 einen Mitnehmer 30 auf, der sich vor der ersten Betätigung in Anlage mit der Innenseite der Bodenfläche 35 befindet. Durch eine Einkerbung weist der Mitnehmer 30 eine radiale Vorspannung auf, die ihn radial in Richtung auf die Innenhülse 29, die an der Bodenfläche 35 des Betätigungsdrückers 22 angeformt ist und eine dem Betätigungsweg s entsprechende Länge aufweist, beaufschlagt.

Die Anzahl n der Teilhübe, in denen das im Spritzenzylinder 11 vorgehaltene Medium ausgebracht wird, beträgt bei der dargestellten Vorrichtung zwei. Somit ist auch das zur Verfügung stehende Volumen für das Medium im Spritzenzylinder vorgegeben. Das Volumen bestimmt sich aus dem Innendurchmesser des Spritzenzylinders 11 und der Verschiebelänge des Kolbens 12 zwischen seiner Ausgangslage (wie dargestellt) und seiner Endlage, wenn er um die vorgegebene Anzahl n von Betätigungswegen s in Richtung auf den Ausbringungskanal 13 hin verschoben wurde. Es ist auch möglich, daß die einzelnen Betätigungselemente unterschiedliche Betätigungswege aufweisen. Dies ist über den Abstand des Mitnehmers 30 zu der Bodenfläche 35 vor dem ersten und zu der Innenhülse 36 vor dem zweiten Betätigungshub möglich. Insbesondere kann, um gleiche Ausbringungsvolumina sicherzustellen, der erste Teilhub größer sein als der zweite Teilhub, da während des ersten Teilhubes ein Leerweg zum Öffnen des Dichtelements 17 erforderlich ist. Im dargestellten Beispiel kann dies dadurch erreicht werden, daß die Innenhülse 29 um ein vorgegebenes Maß länger ist als der Betätigungsweg s des Betätigungsdrükkers 29 beim ersten Teilhub.

Wird der Betätigungsdrücker 22 nunmehr betätigt, was in der Regel dadurch geschieht, daß der Betätigungsdrücker 22 auf der Außenseite der Bodenfläche 35 durch den Daumen des Benutzers mit einer Mindestkraft beaufschlagt wird, so wird der Betätigungsdrücker 22 um den Betätigungsweg s von der Betätigungsausgangslage - wie dargestellt - in die Betätigungsendlage verschoben. Über den Mitnehmer 30 wird der Stößel 14 beaufschlagt und um den gleichen Betätigungsweg s verschoben, wodurch in dem Spritzenzylinder 11 der Kolben 12 um den Betätigungsweg s verschoben wird. Ein dem dabei verdrängten Volumen entsprechendes Volumen an Medium wird aus dem Spritzenzylinder 11 durch den Ausbringungskanal 13 hindurch über die Kappe mit der Düse 31 hinaus ausgebracht und üblicherweise durch die Düse 31 zerstäubt. Zusätzlich zu ihrer Vorspannung wird dabei die Rückstellfeder 26 durch Verkürzung ihrer Länge weiter gespannt. Nach Beendigung der Betätigung wird der Betätigungsdrücker 22 losgelassen. Durch die zwischen Spritzenzylinder 11 und Betätigungsdrücker 22 aufgebrachte Kraft der Rückstellfeder 26 wird der Betätigungsdrücker 22 in die Betätigungsausgangslage zurück verbracht. Durch Formgebung des Kolbens 12 und durch die Wirkung der Rastmittel 27, die in den Spritzenzylinder 11 hineingedrückt wurden, kann sichergestellt werden, daß ein Zurückbewegen des Stößels 14 zusammen mit dem Betätigungsdrücker 22 nicht stattfindet. Vielmehr gleiten die Mitnehmer 30 entlang der Innenhülse 29. Sobald der Betätigungsdrücker 22 wieder die Betätigungsausgangslage, die durch den Abschlußring 25 definiert wird, erreicht, verlassen die Mitnehmer 30 die Innenhülse 29 und gelangen in Anlage mit der Innenhülsenoberkante 36. Bei der nächsten Betätigung wird die Betätigungskraft, die auf den Betätigungsdrücker 22 einwirkt, über die Innenhülse 29 auf die Mitnehmer 30 des Stößels 14 übertragen.

Ein (geringfügiges) Spiel zwischen Innenhülsenoberkante 36 und Mitnehmer 30 stellt einersetis sicher, daß die Mitnehmer 30 tatsächlich die Innenhülse 29 verlassen und ermöglicht andererseits erwünschte Hubwegdifferenzen der ersten und der zweiten Betätigung.

Um eine vorteilhafte große Widerverwertbarkeit von Teilen zu erreichen, kann der Betätigungsdrücker 22 durch Aufbringen einer radial wirkenden Kraft von dem Gehäuse 15 gelöst werden, in dem die Rastnasen 24 soweit in Richtung auf den Stößel 14 hineingedrückt werden, daß sie den Abschlußring 25 nicht mehr hintergreifen. Danach kann der Betätigungsdrücker 22 axial nach hinten abgezogen werden. Die zu diesem Zweck vorzugsweise an der Bodenfläche 35 des Betätigungsdrückers 22 befestigte Rückstellfeder 26 wird ebenfalls nach hinten abgezogen. Nun kann der Spritzenzylinder 11 aus dem Gehäuse 15 entfernt werden. Vorzugsweise wird gleichzeitig die Kappe 16 entfernt. Sofern der Stößel 14 nicht mit dem Kolben 12 verbunden ist, kann auch der Stößel 14 vom Spritzenzylinder herausgenommen werden und erneut verwendet werden. Ein ggf. mit einer Kappe 16 und Dichtelement 17 versehener Spritzenzylinder 11 mit dem darin befindlichen Kolben 12 und neuem, auszubringenden Medium kann nun in das Gehäuse 15 eingesetzt werden. Danach wird unter Erzeugen einer Vorspannung an der Rückstellfeder 26 der Betätigungsdrücker 22 wieder in den Schaft 33 des Gehäuses 15 eingesetzt. Sobald der Betätigungsdrücker 22 wieder durch den Abschlußring 25 gehalten wird, kann durch die Einführungsöffnung 34 des Betätiungsdrückers 22 hindurch der Stößel 14 wieder eingesetzt werden. Diese Vorgehensweise hat den Vorteil, daß der Stößel 14 nicht beim Wiederansetzen des Betätigungsdrückers 22 an dem Schaft 33 zu weit in Richtung des Ausbringungskanals 13 verschoben wird und so unbeabsichtigt ein Teil des auszubringenden Mediums schon vor der ersten Betätigung ausgebracht wird. Im Hinblick auf die Sicherung der Vorrichtung vor unbeabsichtigter Betätigung, kann es auch von Vorteil sein, den Stößel 14 erst unmittelbar vor einer weiteren Benutzung der Vorrichtung wieder durch die Einführungsöffnung 34 hindurch in das Gehäuse 15 und den Spritzenzylinder 11 hin einzusetzen, da ohne ihn ein unbeabsichtigtes Betätigen der Vorrichtung nicht möglich ist.

Die Fig. 2 zeigt ebenfalls einen Querschnitt durch eine Vorrichtung zum zerstäubten Ausbringen eines insbesondere flüssigen Mediums. Bei dieser Vorrichtung wird ebenfalls das Medium in dem Spritzenzylinder 11 bereitgehalten. Der Spritzenzylinder 11 mündet in dem Ausbringungskanal 13 mit seiner Mündung 32, die von dem in der Kappe 16 eingebrachten Dichtelement 17 zur Düse 31 hin verschlossen wird. Die Kappe 16 wird durch die Überwurfkante 18 des Gehäuses 15 am Spritzenzylinder gehalten. Der Spritzenzylinder 11 ist ebenfalls im Gehäuse 15 geführt. Auf der dem Ausbringungskanal 13 abgewandten Seite des Spritzenzylinders 11 ist der Kolben 12 axial verschiebbar angeordnet. Der Kolben 12 wird mittels des Stößels 14 und dem Betätigungsdrücker 22 manuell betätigt. Zur Aufbringung einer Kraft auf den Betätigungsdrücker 22 weist das Gehäuse 15 die Fingeranlage 19 auf. Der Spritzenzylinder 11 ist in dem Gehäuse 15 mit seiner Aufweitung 21 an der Anlagefläche 20 des Gehäuses 15 abgestützt. An der Aufweitung 21 des Spritzenzylinders 11 liegt die Rastscheibe 37 an. Zwischen Rastscheibe 37 - oder Aufweitung 21 - und der Bodenfläche 35 des Betätigungsdrückers 22 ist die Rückstellfeder 26, die für die Rückführung des Betätigungsdrückers 22 aus der Betätigungsendlage in die dargestellte Betätigungsausgangslage sorgt, angeordnet. Der Betätigungsdrücker 22 ist wiederum napfförmig ausgebildet und wird in dem Schaft 33 des Gehäuses 15 geführt. In der dargestellten Betätigungsausgangslage wird der Betätigungsdrücker 22 über nicht dargestellte Mittel am Schaft 33 so gehalten, daß er - vom Ausbringungskanal 13 her gesehen - nicht weiter nach hinten wegbewegt werden kann. Der napfförmige Betätigungsdrücker 22 weist auf seiner Bodenfläche 35 die Einführungsöffnung 34 auf, durch die hindurch der Stößel 14 in den Spritzenzylinder 11 so einführbar ist, daß er in Anlage an den Kolben 12 gelangt. In dieser Lage liegen die vordersten Mitnehmer 30 an der Rastscheibe 37 an. Die nächsten Mitnehmer 30, die von den vorhergehenden Mitnehmern 30 um die Länge s eines Teilhubes entfernt sind, werden von den Schubstegen 38 der Innenhülse 29 des Betätigungsdrückers 22 hintergriffen. Um unterschiedlich große Teilhübe zu ermöglichen, können die Mitnehmer 30 auch einen gegenüber der Länge s geringeren Abstand aufweisen, der jedoch größer sein muß als s/2, damit nicht der übernächste Mitnehmer 30 hintergriffen wird. In diesem Fall gleiten die Schubstege 38 während eines Leerweges entlang dem Stößel 14 bis die kraftschlüssige Verbindung zu dem Mitnehmer 30 des entsprechenden Ausbringungshubes entsteht. Die Innenhülse 29 umgibt die Einführungsöffnung 34 und ragt von der Bodenfläche 35 des Betätigungsdrückers 22 in Richtung auf den Spritzenzylinder 11 ab. Die Innenhülse 29 weist Schubstege 38 auf, die in den Bewegungsraum des Stößels 14 hineinragen, jedoch so elastisch sind, daß sie während des Rückhubes des Betätigungsdrückers 22 von der Betätigungsendlage in die Betätigungsausgangslage soweit radial nach außen gedrückt werden können, daß sie über die Mitnehmer 30 des Stößels 14 hinweg bewegt werden können und so den Bewegungsraum für die Relativbewegung zwischen Schaft 14 und Betätigungsdrücker 22 während des Rückhubes freigeben.

Wird der Betätigungsdrücker 22 betätigt, so muß zunächst eine solche Kraft aufgebracht werden, daß der vorderste Mitnehmer 30, der an der Rastscheibe 37 anliegt, die eine Rastkante bildet, die Rastscheibe 37 reversibel so spreizt, daß der Stößel 14 in den Spritzenzylinder 11 hineinbewegt werden kann. Ist diese Mindestbetätigungskraft überschritten, so wird der Schaft 14, von den Schubstegen 38 des Betätigungsdrückers 22 geschoben, weiter in den Spritzenzylinder 11 in Richtung auf den Ausbringungskanal 13 hineinbewegt und schiebt dabei den Kolben 12 des Spritzenzylinders 11 mit. Dies geschieht soweit, bis der Betätigungsdrücker 32 mit seiner dem Spritzenzylinder 11 zugewandten Oberkante an der Aufweitung 21 des Spritzenzylinders anliegt. In dieser Lage sind die ersten Mitnehmer 30 in den Spritzenzylinder 11 hineinbewegt, die zweiten Mitnehmer 30 liegen an der Rastscheibe 37 an, die Rückstellfeder 26 ist komprimiert. Wird der Betätigungsdrücker 22 nun losgelassen, so sorgt die Rückstellfeder 26 dafür, daß er wieder in die dargestellte Betätigungsausgangslage zurückbewegt wird. Dabei streifen die Schubstege 38 entlang dem Stößel 14, bis sie von der Vorderkante der dritten Mitnehmer 30 gespreizt werden und so über diese hinweg bewegt werden. Sobald die Schubstege 38 über die Mitnehmer 30 hinwegbewegt sind, legen sie sich wieder an den Stößel 14 an und hintergreifen die dritten Mitnehmer 30. Die zweiten Mitnehmer 30 dienen für den zweiten und im dargestellten Beispiel letzten Teilhub als Rastmittel, die das Aufbringen der erforderlichen Mindestbetätigungskraft für den weiteren Teilhub sicherstellen.

Im dargestellten Ausführungsbeispiel sind die Mitnehmer 30 als Verdickungen, d.h. als radiale Querschnittserweiterungen, des Stößels 14 ausgebildet. Der Stößel 14 weist im Bereich der Mitnehmer 30 einen kegelstumpfförmigen Querschnitt auf, wobei die Kegelspitze in Richtung des Kolbens 12 des Spritzenzylinders 11 zeigt. Die Schubstege 38 liegen zur Kraftübertragung auf den Stößel 14 an der Bodenfläche des Kegelstumpfes der Mitnehmer 30 an.

Um eine größere Anzahl von Teilhüben zu ermöglichen, ist es lediglich erforderlich, den Stößel 14 nach hinten zu verlängern und ihn mit weiteren Mitnehmern 30 zu versehen. Gleichzeitig muß der mögliche Weg des Kolbens 12 im Spritzenzylinder 11 einen entsprechenden Weg zurücklegen können.

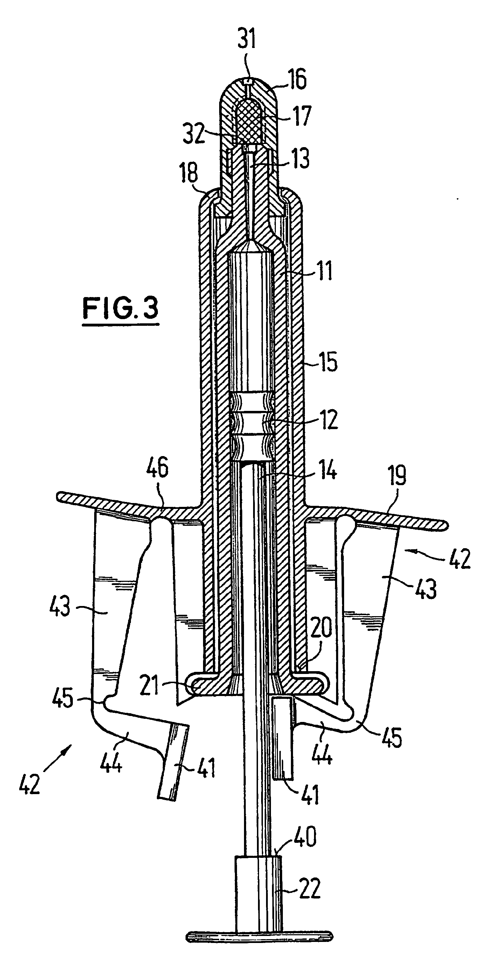

### AUSFÜHRUNGSBEISPIEL NACH FIG. 3

Die Fig. 3 zeigt in einem Querschnitt die Darstellung einer Vorrichtung zum ggf. zerstäubten Ausbringen eines insbesondere flüssigen Mediums, bei dem die Unterteilung des Betätigungsweges des Kolbens des Spritzenzylinders in Teilhübe mittels Gleitschuhen erfolgt.

Ein Spritzenzylinder 11 weist an seinem vorderen Ende den Ausbringungskanal 13 auf. Dieser mündet in der Kappe 16, wobei die Kappe 16 eine Düse 31 aufweist und wobei der Raum zwischen Düse 31 und Mündung 32 des Ausbringungskanals 13 durch das Dichtelement 17 verschlossen ist. Der Spritzenzylinder 11 und die Kappe 16 sind in dem Gehäuse 15 durch die Überwurfkante 18 gehalten. In dem Spritzenzylinder 11 ist der Kolben 12 axial verschiebbar gelagert. An dem Kolben 12 ist der Stößel 14, der nach hinten aus dem Spritzenzylinder 11 hinausragt, befestigt. Das Gehäuse 15 erstreckt sich über die Länge des Spritzenzylinders 11, so daß die Aufweitung 21 des Spritzenzylinders 11 an der Anlagefläche 20 des Gehäuses 15 klemmend gehalten werden.

Der an dem Kolben 12 des Spritzenzylinders 11 angeformte Stößel 14 weist auf seinem dem Kolben 12 abegwandten, hinteren Ende einen Bund 40 auf, der durch eine Querschnittserweiterung des Stößels 14 gebildet wird. Die Querschnittserweiterung mündet im dargestellten Fall in dem Betätigungsdrücker 22, der das Aufbringen einer Betätigungskraft durch den Benutzer auf den Stößel 14 erlaubt.

An dem Gehäuse 15 sind seitlich abragende Fingeranlagen 19 angeformt. Dabei weist die Fingeranlage 19 in ihrem Befestigungsbereich an dem Gehäuse 15, das den Spritzenzylinder 11 ummantelt, ein Scharnier - insbesondere ein Filmscharnier 46 - auf. Von der Fingeranlage 19, radial gesehen außerhalb des Filmscharniers, ragt ein Hebel 42 nach hinten ab. Der Hebel 42 wird aus einem ersten Teilarm 43, der sich im wesentlichen in axialer Richtung nach hinten erstreckt und einem zweiten Teilarm 44, der mit dem ersten Teilarm 43 über ein Gelenk 45, insbesondere ein Filmscharnier, verbunden ist, gebildet und weist an seinem anderen Ende den Gleitschuh 41 auf.

In der Figur ist auf der linken Figurenhälfte die Lage dargestellt, die sich ergibt, wenn keine Handkraft auf die Fingeranlage 19 ausgeübt wird; in der rechten Bildhälfte ist der Fall gezeigt, wenn eine Handkraft auf die Fingeranlage 19 einwirkt.

Gemäß der Darstellung auf der linken Seite der Figur ist das Filmscharnier 46 und das Gelenk 45 derart vorgespannt, daß der Gleitschuh 41 nicht an dem Stößel 41 anliegt, solange keine Kraft auf die Fingeranlage 19 aufgebracht wird. Dies hat den Vorteil, daß in einfacher Weise der Spritzenzyilnder 11, ggf. mit der daran befestigten Kappe 16, und der Kolben 12 mit dem Stößel 14 samt im Betätigungsdrücker 22 vor der Betätigung in das Gehäuse 15 eingesetzt und im Gehäuse 21 durch die Formgebung der Anlagefläche 20 gehalten werden kann. Ebenso einfach ist dann nach dem letzten Teilhub das Entfernen des benutzen Spritzenzylinders 11. Das Gehäuse 15 sowie die daran angeformte Fingeranlage 19 mitsamt den Hebeln 42 und den Gleitschuhen 41 kann für eine Vielzahl von Benutzungen wieder verwendet werden, während der Spritzenzylinder 11 mitsamt der Kappe 16 und dem Kolben 12 sowie dem Stößel 14 und dem Betätigungsdrücker 22, beispielsweise aus hygienischen Gründen, jeweils nur eine Verwendung erfolgt.

Wird auf die Fingeranlage 19 ein Finger aufgelegt und dadurch eine Kraft aufgebracht, so wird die Fingeranlage 19 in dem Bereich der radial außerhalb des Filmscharniers 46 liegt, nach hinten (vom Ausbringungskanal 13 abgewandte Seite) gedrückt. Die Hebel 42 mit ihren ersten Teilarmen 43 und zweiten Teilarmen 44 bringen die Gleitschuhe 41 in Anlage an den Strößel 14. Wird der Betätigungsdrücker nun betätigt, so gleiten die Gleitschuhe solange an dem Stößel 14 entlang, bis der Bund 40 in Anlage an die Gleitschuhe 40 gelangt und ein Verklemmen zwischen Bund 40, Stößel 14 und Gleitschuhen 41 erfolgt. Dadurch wird verhindert, daß der Stößel 14 weiter als bis zum Bund 40 in Richtung auf den Aufbringungskanal 13 nach vorne geschoben wird. Der Bund 40 besteht in einer Querschnittserweiterung des Stößels 14. Mit seiner Vorderkante gelangen die Gleitschuhe 41 beim Verklemmen vorzugsweise in Anlage an die Aufweitung 21 des Spritzenzylinders 11, wodurch die Gleitschuhe weiter abgestützt werden. Das vorgespannte Gelenk 45 zwischen dem ersten Teilarm 43 und dem zweiten Teilarm 44 des Hebels 42 wirkt als Feder, die nach Entlastung des Betätigungsdrückers 22 dafür sorgt, daß die Gleitschuhe axial gegen die Hubrichtung über den Bund 40 geschoben werden. Dadurch wird verhindert, daß beim erneuten Betätigen des Betätigungsdrückers 22 ein erneutes Einrasten an demselben Bund 40 erfolgt. Durch mehrfache Anordnung eines Bundes 40 am Stößel 14 können mit diesem Prinzip auch mehrere definierte Hübe erzeugt werden. Über unterschiedliche Abstände eines Bundes 40 zu einem darauffolgenden können definierte Hübe unterschiedlicher Größe ermöglicht werden.

## Patentansprüche

1. Vorrichtung zum ggf. zerstäubten Ausbringen eines insbesondere flüssigen Mediums, mit einem Spritzenzylinder (11), in dem einem darin verschiebbaren Kolben (12) und einem Ausbringungskanal (13), über den das Medium ausbringbar ist, wobei der Kolben (12) über einen Stößel (14) manuell betätigbar ist und wobei an dem Stößel (14) ein Betätigungsdrücker (22) angreift und wobei der Betätigungsweg (s) zwischen Betätigungsausgangslage und Betätigungsendlage des Betätigungsdrückers (22) so begrenzt ist, dass der aus einer Betätigung des Betätigungsdrückers (22) resultierende Ausbringungshub (s) des Kolbens (12) auf das für das Ausbringen einer Teilcharge benötigte Maß begrenzt ist,
**dadurch gekennzeichnet, dass** an dem Stößel (14) in den Teilhüben entsprechendem Abstand (s) Mitnehmer (30) angeordnet sind, wobei diese Mitnehmer (30) lediglich in Richtung des Ausbringungshubes eine Mitnahmeverbindung zwischen Betätigungsdrücker (22) und Stößel (14) herstellen.

2. Vorrichtung nach Anspruch 1, **dadurch gekennzeichnet, dass** eine Rückstellfeder (26) so angeordnet ist, dass der Betätigungsdrücker selbsttätig in die Betätigungsausgangslage zurückgeführt wird.

3. Vorrichtung nach Anspruch 1 oder 2, **dadurch gekennzeichnet, dass** der Betätigungsweg (s) des Betätigungsdrückers (22) auf das für den größten Ausbringungshub (s) erforderliche Maß begrenzt ist.

4. Vorrichtung nach einem der Ansprüche 1 bis 3, **dadurch gekennzeichnet, daß** an dem Stößel (14) Rastmittel (27) angeformt sind, die in Betätigungsausgangslage an einer Rastkante (28) anliegen und die durch Überwinden einer Mindestbetätigungskraft überdrückbar sind, wobei vorzugsweise die Mitnehmer (30) so ausgebildet sind, daß der Mitnehmer (30) eines Ausbringungshubes als Rastmittel eines nachfolgenden Ausbringungshubes dient.

5. Vorrichtung nach einem der Ansprüche 3 bis 6, **dadurch gekennzeichnet, daß** die Mitnehmer (30) als Verdickungen des Stößels und insbesondere aus kegelstumpfförmigen Abschnitten des Stößels (14) gebildet sind, deren imaginäre Kegelspitze in Richtung des Kolbens (12) weist.

6. Vorrichtung nach einem der Ansprüche 1 bis 5, **dadurch gekennzeichnet, daß** der Spritzenzylinder (11) in einem Gehäuse (15) gehalten ist, wobei das Gehäuse (15) vorzugsweise angeformte Fingerauflagen (19) für wenigstens einen Finger aufweist.

7. Vorrichtung nach Anspruch 6, **dadurch gekennzeichnet, daß** der Betätigungsdrücker (22) im Gehäuse (15) geführt und insbesondere lösbar an dem Gehäuse (15) angeordnet ist.

8. Vorrichtung nach einem der Ansprüche 6 oder 7, **dadurch gekennzeichnet, daß** das Gehäuse (15) eine Anlagefläche (20) aufweist, an der eine Aufweitung (21) des Spritzenzylinders (11) in Anlage bringbar ist, wodurch eine Abstützung des Spritzenzylinders (11) gegen die Betätigungskraft des Stößels (14) erfolgt, wobei die Aufweitung (21) vorzugsweise am dem Ausbringungskanal (13) abgewandten Ende des Spritzenzylinders (11) ausgebildet ist.

9. Vorrichtung nach Anspruch 8, **dadurch gekennzeichnet, daß** auf der Aufweitung (21) eine Rastscheibe (37) aufliegt, an der die Rückstellfeder (26) abgestützt ist, wobei vorzugsweise die Rastscheibe (37) als Rastkante (28) dient.

10. Vorrichtung nach dem Oberbegriff von Anspruch 1, **dadurch gekennzeichnet, daß** wenigstens ein Gleitschuh (41) an dem Stößel (14) in einem Bereich außerhalb des Spritzenzylinders (11) anliegt und daß der Stößel (14) wenigstens einen Bund (40) aufweist, wobei in Betätigungsendlage eines Betätigungshubes ein Bund (40) in Anlage mit dem wenigstens einen Gleitschuh (41) gelangt.

11. Vorrichtung nach Anspruch 10, **dadurch gekennzeichnet, daß** der wenigstens eine Gleitschuh (41) mit seiner dem wenigstens einen Bund (40) abgewandten Seite am Spritzenzylinder (11) abgestützt ist und daß der wenigstens eine Bund (40) insbesondere durch eine Querschnittsvergrößerung des Stößels (14) gebildet wird.

12. Vorrichtung nach einem der Ansprüche 10 oder 11, **dadurch gekennzeichnet, daß** der wenigstens eine Gleitschuh (41) über einen Hebel (42) an der am Gehäuse (15) ausgebildeten Fingerauflage (19) angelenkt sind, wobei erst durch das Aufbringen einer Stützkraft an der Fingerauflage (19) der Gleitschuh (41) in Anlage an den Stößel (14) gerät, wobei daß der Hebel (42) vorzugsweise aus zwei Teilarmen (43, 44) besteht, die untereinander über ein Gelenk (45) miteinander verbunden sind, wobei das Gelenk (45) derart vorgespannt ist, daß bei einer Entlastung des Hebels (42) eine der Betätigungsrichtung des Stößels entgegengesetzte axiale Bewegung des Gleitschuhs (41) erzeugt wird.

13. Vorrichtung nach einem der Ansprüche 1 bis 12, **dadurch gekennzeichnet, daß** der Ausbringungskanal (13) in einer Kappe (16) mündet, wobei die Kappe (16) eine Austrittsöffnung, insbesondere eine Düse (31), aufweist.

14. Vorrichtung nach Anspruch 13, **dadurch gekennzeichnet, daß** der Strömungsweg zwischen Ausbringungskanal (13) und Austrittsöffnung über ein Dichtelement (17) verschlossen ist, wobei der Strömungsweg vorzugsweise aufgrund des Mediendrucks während der Betätigung des Betätigungsdrükkers (22) reversiebel freigebbar ist.

15. Vorrichtung nach Anspruch 14, **dadurch gekennzeichnet, daß** der Strömungsweg irreversibel bei der ersten Betätigung des Betätigungsdrückers (22) freigegeben wird.

16. Vorrichtung nach einem der Ansprüche 13 bis 15, **dadurch gekennzeichnet, daß** die Kappe (16) über einen Preßsitz am Ausbringungskanal (13) befestigt ist.

17. Vorrichtung nach einem der Ansprüche 13 bis 16, **dadurch gekennzeichnet, daß** die Kappe (16) im Gehäuse insbesondere an einer Überwurfkante (18) gehalten wird.

18. Vorrichtung nach einem der vorhergehenden Ansprüche, **dadurch gekennzeichnet, daß** zumindest der Spritzenzylinder (11) mit seinem Kolben (12) austauschbar im Gehäuse (15) gehalten ist, wobei vorzugsweise zusätzlich wenigstens eines der folgenden Elemente: Stößel (14), Kappe (16) und Dichtelement (17) austauschbar ist.

19. Vorrichtung nach einem der vorhergehenden Ansprüche, **dadurch gekennzeichnet, dass** Mitnehmer (30) in Teilhüben unterschiedlicher Größe entsprechendem Abstand angeordnet sind.

## Claims

1. Apparatus for the optionally atomized discharge of an in particular liquid medium, having a spray cylinder (11) in which a piston (12) is displaceably located, and a discharge channel (13) by means of which the medium can be discharged, the piston (12) being manually operable by means of a ram (14) on which acts an operating trigger (22) and in which the operating path(s) between the operating starting position and the operating end position of the operating trigger (22) is limited in such a way that the discharge stroke(s) of the piston (12) resulting from the operation of the operating trigger (22) is limited to the amount required for the discharge of a partial charge, **characterized in that** on the ram (14) with the spacing(s) corresponding to the partial strokes are provided drivers (30) which only produce a driving connection between the operating trigger (22) and the ram (14) in the discharge stroke direction.

2. Apparatus according to claim 1, **characterized in that** a return spring (26) is positioned in such a way that the operating trigger is automatically returned to the operating starting position.

3. Apparatus according to claim 1 or 2, **characterized in that** the operating path(s) of the operating trigger (22) is limited to the amount necessary for the maximum discharge stroke(s).

4. Apparatus according to one of the claims 1 to 3, **characterized in that** on the ram (14) are shaped locking means (27), which in the operation starting position engage on a locking edge (28) and which are overpressable by overcoming a minimum operating force, the drivers (30) preferably being constructed in such a way that the driver (30) of one discharge stroke serves as the locking means for a following discharge stroke.

5. Apparatus according to one of the claims 3 and 4, **characterized in that** the drivers (30) are constructed as thickenings of the ram and in particular are formed from frustum-shaped sections of the ram (14), whose imaginary cone apex points in the direction of the piston (12).

6. Apparatus according to one of the claims 1 to 5, **characterized in that** the spray cylinder (11) is held in a casing (15), which has finger bearing surfaces (19) for at least one finger.

7. Apparatus according to claim 6, **characterized in that** the operating trigger (22) is guided in the casing (15) and is in particular detachably placed on the casing (15).

8. Apparatus according to one of the claims 6 or 7, **characterized in that** the casing (15) has a bearing surface (20) on which can be engaged a widening (21) of the spray cylinder (11), so that the spray cylinder (11) is supported against the operating force of the ram (14), the widening (21) preferably being formed on the end of the spray cylinder (11) remote from the discharge channel (13).

9. Apparatus according to claim 8, **characterized in that** a locking disk (37), on which the return spring (26) is supported, rests on the widening (21), the locking disk (37) preferably serving as a locking edge (28).

10. Apparatus according to the preamble of claim 1, **characterized in that** at least one sliding shoe (41) engages on the ram (14) in an area outside the spray cylinder (11) and wherein the ram (14) has at least one collar (40) and in the operating end position of an operating stroke a collar (40) engages with the at least one sliding shoe (41).

11. Apparatus according to claim 10, **characterized in that** at least one sliding shoe (41) is supported with its side remote from the at least one collar (40) on the spray cylinder (11) and that the at least one collar (40) is in particular formed by a cross-sectional enlargement of the ram (14).

12. Apparatus according to one of the claims 10 or 11, **characterized in that** the at least one sliding shoe (41) is articulated by means of a lever (42) to the finger bearing surface (19) constructed on the casing (15) and only through the application of a supporting force to the finger bearing surface (19) does the sliding shoe (41) engage with the ram (14), the lever (42) preferably comprising two partial arms (43, 44), which are interconnected by means of a joint (45), which is so biased that on releasing the lever (42) an axial movement of the sliding shoe (41) is produced which opposes the operating direction of the ram.

13. Apparatus according to one of the claims 1 to 12, **characterized in that** the discharge channel (13) issues into a cap (16), which has an outlet port, particularly a nozzle (31).

14. Apparatus according to claim 13, **characterized in that** the flow path between the discharge channel (13) and the outlet port is sealed by means of a sealing element (17), the flow path preferably being reversibly freed as a result of medium pressure during the operation of the operating trigger (22).

15. Apparatus according to claim 14, **characterized in that** the flow path is irreversibly freed during the first operation of the operating trigger (22).

16. Apparatus according to one of the claims 13 to 15, **characterized in that** the cap (16) is fixed by a press fit to the discharge channel (13).

17. Apparatus according to one of the claims 13 to 16, **characterized in that** the cap (16) is held in the casing, particularly on a connecting edge (18).

18. Apparatus according to one of the preceding claims, **characterized in that** at least the spray cylinder (11) with its piston (12) is interchangeably held in the casing (15) and preferably additionally at least one of the following elements is replaceable: ram (14), cap (16) and sealing element (17).

19. Apparatus according to one of the preceding claims, **characterized in that** drivers (30) with spacings corresponding to partial strokes of differing magnitude are provided.

## Revendications

1. Dispositif pour la décharge sous forme éventuellement nébulisée d'une substance notamment liquide, avec un cylindre de seringue (11) dans lequel peut être déplacé un piston (12) et avec un conduit de décharge (13) par lequel peut être déchargée la substance, sachant que le piston (12) peut être actionné manuellement par une tige-poussoir (14) et sachant qu'un poussoir d'actionnement (22) s'engage sur la tige-poussoir (14) et sachant que la trajectoire d'actionnement (s) entre la position initiale d'actionnement et la position terminale d'actionnement du poussoir d'actionnement (22) est limitée de telle manière que la course de décharge (s) du piston (12), qui résulte de l'actionnement du poussoir d'actionnement (22), est limitée à la mesure indispensable à la décharge d'une dose partielle, **caractérisé en ce que** des d'entraîneurs (30) sont disposés sur la tige-poussoir (14) avec un écart (s) qui correspond aux courses partielles, sachant que ces entraîneurs (30) créent un raccord d'entraînement entre le poussoir d'actionnement (22) et la tige-poussoir (14) uniquement en direction de la course de décharge.

2. Dispositif d'après la revendication 1, **caractérisé en ce qu'**un ressort de rappel (26) est disposé de telle manière que le poussoir d'actionnement est ramené automatiquement dans la position initiale d'actionnement.

3. Dispositif d'après la revendication 1 ou 2, **caractérisé en ce que** la trajectoire d'actionnement (s) du poussoir d'actionnement (22) est limitée à une mesure imposée par une course de décharge (s) maximale.

4. Dispositif d'après une des revendications de 1 à 3, **caractérisé en ce que** des moyens d'enclenchement (27) sont ajoutés par moulage à la tige-poussoir (14) et qui font suite, dans leur position initiale d'actionnement, à une arête d'arrêt (28) et qui peuvent être franchis en surmontant une force minimale d'actionnement, sachant que les entraîneurs (30) sont réalisés de préférence de telle manière qu'un entraîneur (30) d'une course de décharge puisse servir de moyen d'enclenchement pour une course de décharge ultérieure.

5. Dispositif d'après une des revendications de 3 à 4, **caractérisé en ce que** les entraîneurs (30) sont réalisés en tant que renflements de la tige-poussoir et notamment en tant que sections de forme tronconique de la tige-poussoir (14) dont la pointe fictive du cône est orientée en direction du piston (12).

6. Dispositif d'après une des revendications de 1 à 5, **caractérisé en ce que** le cylindre de seringue (11) est tenu dans un boîtier (15), sachant que le boîtier (15) présente des appuis pour les doigts (19), formés de préférence par moulage, pour au moins un doigt.

7. Dispositif d'après la revendication 6, **caractérisé en ce que** le poussoir d'actionnement (22) est conduit dans le boîtier (15) et qu'il est disposé sur le boîtier (15) de manière notamment détachable.

8. Dispositif d'après une des revendications 6 ou 7, **caractérisé en ce que** le boîtier (15) présente une surface de contact (20) contre laquelle un élargissement (21) du cylindre de seringue (11) peut être mise en contact, ce qui exerce une action de support du cylindre de seringue (11) contre la force d'actionnement de la tige-poussoir (14), sachant que l'élargissement (21) est réalisé de préférence à l'extrémité du cylindre de seringue (11) opposée au conduit de décharge (13).

9. Dispositif d'après la revendication 8, **caractérisé en ce que** sur l'élargissement (21) repose un disque d'arrêt (37), contre lequel s'appuie le ressort de rappel (26), le disque d'arrêt (37) servant de préférence d'arête d'arrêt (28).

10. Dispositif d'après le préamble de la revendication 1, **caractérisé en ce qu'**au moins un patin de guidage (41) est adjacent à la tige-poussoir (14) dans un domaine à l'extérieur du cylindre de seringue (11) et **en ce que** la tige-poussoir (14) présente au moins un épaulement (40), sachant qu'en position terminale d'actionnement d'une course d'actionnement un épaulement (40) devient adjacent au ou aux patins de guidage (41).

11. Dispositif d'après la revendication 10, **caractérisé en ce que** le ou les patins de guidage (41) sont soutenus par le cylindre de seringue (11) par sa face opposée à le ou les épaulements (40) et **en ce que** le ou les épaulements (40) est notamment constitué d'un agrandissement de section transversale de la tige-poussoir (14).

12. Dispositif d'après une des revendications 10 ou 11, **caractérisé en ce que** le ou les patins de guidage (41) sont fixés de manière articulée par un levier (42) à l'appui pour les doigts (19) réalisé sur le boîtier (15), sachant que le patin de guidage (41) devient adjacent à la tige-poussoir (14) uniquement si on fournit une force de soutien à l'appui pour les doigts (19), **en ce que** le levier (42) est composé de préférence de deux bras partiels (43, 44) raccordés entre eux par une articulation (45), l'articulation (45) se trouvant sous précontrainte de telle manière que quand le levier (42) est déchargé est produit un mouvement axial du patin de guidage (41) opposé à la direction d'actionnement de la tige-poussoir.

13. Dispositif d'après une des revendications de 1 à 12, **caractérisé en ce que** le conduit de décharge (13) débouche dans un capuchon (16), sachant que le capuchon (16) présente un orifice de sortie, notamment une buse (31).

14. Dispositif d'après la revendication 13, **caractérisé en ce que** la voie de l'écoulement entre le conduit de décharge (13) et l'orifice de sortie est close au moyen d'un élément d'étanchement (17), la voie de l'écoulement pouvant être débloquée de manière réversible pendant l'actionnement du poussoir d'actionnement (22), de préférence en raison de la pression de la substance.

15. Dispositif d'après la revendication 14, **caractérisé en ce que** la voie de l'écoulement est débloquée de manière irréversible pendant le premier actionnement du poussoir d'actionnement (22).

16. Dispositif d'après une des revendications de 13 à 15, **caractérisé en ce que** le capuchon (16) est fixé au conduit de décharge (13) par un ajustement avec serrage.

17. Dispositif d'après une des revendications de 13 à 16, **caractérisé en ce que** le capuchon (16) est maintenu dans le boîtier notamment auprès d'un bord à collerette (18).

18. Dispositif d'après une des revendications précédentes, **caractérisé en ce qu'**au moins le cylindre de seringue (11) avec son piston (12) est maintenu de manière remplaçable dans le boîtier (15), sachant que de préférence en outre au moins un des éléments suivants : la tige-poussoir (14), le capuchon (16) et l'élément d'étanchement (17) est remplaçable.

19. Dispositif d'après une des revendications précédentes, **caractérisé en ce que** des entraîneurs (30) sont disposés de manière à présenter un écart correspondant à des courses partielles de dimensions différentes.
